**Europäisches Patentamt**

**European Patent Office**

**Office européen des brevets**

(19)

(11) Veröffentlichungsnummer: **0 070 492**
**B1**

(12) # EUROPÄISCHE PATENTSCHRIFT

(45) Veröffentlichungstag der Patentschrift:
**28.01.87**

(51) Int. Cl.⁴: **C 11 C 3/00,** C 11 B 3/00,
C 07 C 143/90

(21) Anmeldenummer: **82106223.9**

(22) Anmeldetag: **12.07.82**

(54) Verfahren zur Herstellung von Fettsäurealkylestern mit verbesserter Verarbeitbarkeit.

(30) Priorität: **20.07.81 DE 3128646**

(43) Veröffentlichungstag der Anmeldung:
**26.01.83 Patentblatt 83/4**

(45) Bekanntmachung des Hinweises auf die Patenterteilung:
**28.01.87 Patentblatt 87/5**

(84) Benannte Vertragsstaaten:
**AT BE CH DE FR IT LI LU NL SE**

(56) Entgegenhaltungen:
**DE - B - 1 131 835**
**DE - B - 1 214 212**

(73) Patentinhaber: **Henkel Kommanditgesellschaft auf Aktien, Postfach 1100 Henkelstrasse 67, D-4000 Düsseldorf-Holthausen (DE)**

(72) Erfinder: **Schmid, Karl, Dr., Stifterstrasse 10, D-4020 Mettmann (DE)**
Erfinder: **Baumann, Horst, Dr., Vogelwarte 17, D-5672 Leichlingen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Seit Jahrzehnten is bekannt, waschaktive α-Sulfofettsäureester aus Fetten und Ölen, insbesondere natürlichen Ursprungs zu gewinnen. So beschreibt die US-PS 2 195 187 α-Sulfofettsäuren und ihre Ester als waschaktive Substanzen. Sie werden durch Sulfonierung niederer Alkylester gesättigter höherer Fettsäuren mit Schwefeltrioxid erhalten. Die niederen Fettsäurealkylester werden durch Umesterung hydrierter Fette oder Öle mit einwertigen niederen Alkanolen, insbesondere Methanol bzw. durch Glyceridspaltung und anschliessende Veresterung der Fettsäuren gewonnen.

Die Anmelderin hat sich in späteren Arbeiten intensiv mit dieser Klasse der waschaktiven α-sulfonierten Fettsäuren und entsprechenden Fettsäureester sowie deren Salzen auseinandergesetzt. So beschreibt beispielsweise die DE-AS 1 246 718 ein Verfahren zur Herstellung dieser Verbindungsklasse. Fettsäuren und Fettsäureester, die 6–28 C-Atome im Fettsäurerest aufweisen und ausser dem α-ständigen C-Atom des Fettsäurerestes keine weiteren sulfonierbaren bzw. sulfatierbaren Gruppen aufweisen und eine Jod-Zahl unterhalb 5 besitzen, werden mit einem Schwefeltrioxid-Inertgas-Gemisch sulfoniert und das Umsetzungsprodukt neutralisiert. Ein mit letztlich gleichen Mitteln arbeitendes Parallelverfahren ist in der DE-AS 1 248 645 geschildert.

Eine der Hauptschwierigkeiten des hier betroffenen Gebiets liegt in der Farbinstabilität der Fettsäurealkylester im Sulfonierungsschritt. Es werden dunkel gefärbte, braunschwarze Rohprodukte erhalten, die zur Anwendung in Wasch- und Reinigungsmitteln zu hellfarbigen Produkten aufzuarbeiten sind. Die Farbe der Sulfonierungsrohprodukte ist zwar in bestimmtem Ausmass von den Arbeitsbedingungen abhängig, gleichwohl wird bis heute die technische Verwertung dieser an sich interessanten Rohstoffmöglichkeit durch die folgende Gesetzmässigkeit behindert: Je höher die Umsatzausbeute in der Sulfonierungsstufe (der Sulfonierungsgrad) getrieben wird, umso dunkler gefärbt ist das Reaktionsprodukt, und umso grösser werden die Schwierigkeiten, hellfarbige Endprodukte zu gewinnen.

Die Bedeutung der Konstitution der zu sulfonierenden Fettsäure- bzw. Fettsäuregemische gilt in der Fachwelt als gesichertes Wissen. Es wird insbesondere gefordert, dass die in α-Position zu sulfonierenden Fettsäuren keine bzw. möglichst wenig Doppelbindungen sowie keine sonstigen reaktiven Gruppen – insbesondere Hydroxylgruppen – enthalten sollen. Durch Auswahl geeigneter Fette bzw. Öle wird dieses Problem auf die Beseitigung ungesättigter Bindungen im Fettsäuremolekül eingeschränkt. Diese Störstellen werden durch möglichst weitgehende Hydrierung des Ausgangsmaterials vor der Sulfonierung beseitigt. Die Literatur des Standes der Technik fordert Jod-Zahlen unterhalb von 5, vorzugsweise unterhalb von 2. In den praktischen Beispielen wird mit weit niedrigeren Jod-Zahlen, z.B. solchen im Bereich von 0,1–0,3 gearbeitet.

Auch die destillative oder andersartige Abtrennung störender Begleitstoffe von den zu sulfonierenden Fettsäuren bzw. Fettsäureestern wird zur Verringerung des Verfärbungsproblems gefordert, siehe beispielsweise DE-AS 1 248 645.

Stets ist jedoch als abschliessender Verfahrensschritt eine Bleiche der Rohsulfonsäure-Derivate nötig. Der Stand der Technik hat hier insbesondere 2 Verfahrenstypen entwickelt: Die saure Bleiche mit Wasserstoffperoxid – siehe hierzu DE-PS 1 179 931 – oder eine Kombinationsbleiche, bei der sich an eine zunächst saure Wasserstoffperoxidbleiche die Neutralisierung des solfonierten und teilgebleichten Gutes anschliesst, woraufhin erneut mit Wasserstoffperoxid oder besser mit Hypochlorit eine abschliessende Bleichstufe erfolgt, siehe hierzu beispielsweise DE-AS 1 234 709.

Besondere Schwierigkeiten zum Problem der Verfärbung treten auf, wenn die Sulfonierung auf Umsatzausbeuten über 90% oder gar auf Sulfonierungsgrade über 95% getrieben werden soll. Die Lehre der DE-OS 1 443 995 beschäftigt sich mit den hier entstehenden Problemen. Das Schwefeltrioxid hat nach Angaben dieser Druckschrift auf gesättigte, von alkoholischen Hydroxylgruppen freie Fettsäureestern eine stark zersetzende Wirkung, die bei der Herstellung hochsulfonierter Produkte mit einem Sulfonierungsgrad von wenigstens 90%, vorzugsweise wenigstens 94% und insbesondere wenigstens 96% unmeidbar zu tiefdunkel verfärbten Sulfonierungsrohprodukten führt.

Zur Einschränkung der Verfärbung schlägt die DE-OS 1 443 995 neben der Einhaltung bestimmter Temperaturen vor, in die Sulfonierung bzw. in das Sulfonierungsprodukt Wasser einzubringen. Für die Praxis werden hierdurch neue Schwierigkeiten erzeugt. Die Viskosität des Sulfonierungsrohproduktes wird im stark sauren Bereich schon durch geringste Wassermengen schwerwiegend beeinflusst. Schon der Zusatz von 2% Wasserstoffperoxid in Form einer 35-%igen Lösung führt bei Sulfonierungsprodukten mit der Kettenlänge $C_{16}/C_{18}$ zu einem steilen Viskositätsanstieg. Im kontinuierlichen technischen Verfahren entsteht die Gefahr der Verstopfung von Rohrleitungen.

Die vielgestaltigen und in unterschiedlichen Stufen des Gesamtverfahrens auftretenden Schwierigkeiten führen nach bisherigem Wissen zum aufgezwungenen Kompromiss zwischen Sulfonierung und Bleichung. Die in der Praxis optimal einstellbaren Sulfonierungsgrade liegen bei ca. 90%.

Natürliche Ausgangsmaterialien wie pflanzliche und/oder tierische Fette bzw. Öle unterliegen bekanntlich ganz allgemein gewissen Qualitätsschwankungen – bedingt beispielsweise durch die Besonderheiten ihres Ursprungs und/oder ihrer Handhabung bis zur geplanten Weiterverarbeitung –, die eine gewisse Erschwernis gegenüber standartisierten rein synthetischen Ausgangsmaterialien bedeuten. Es besteht dementsprechend ein Bedürfnis auf dem hier betroffenen engeren Sachgebiet der Fettsäurealkylester durch ein einfaches Raffinationsverfahren die Produktqualität unterschiedlicher Ausgangsmaterialien so zu vereinheitlichen sowie die Verarbeitbarkeit des raffinierten Materials so zu verbessern, dass im grosstechnischen Verfahren sichere und verbesserte Weiterverarbeitungsergebnisse und/oder Produkte mit verbesserten Eigenschaften gewährleistet sind.

Die Erfindung geht von der Aufgabe aus, diesem Bedürfnis zu entsprechen. Sie will damit beispielsweise die geschilderten Probleme der Verfärbung von Fettsäurealkylestern bzw. ihren Gemischen bei der Sulfonierung verringern. Die Erfindung will hier im engeren Sinne eine Vorbehandlung für die zu sulfonierenden Fettsäurealkylester schaffen, durch die eine wirkungsvolle Einschränkung der Verfärbung in der nachfolgenden Sulfonierungsstufe gelingt. Auf der anderen Seite will die Erfindung durch ein einfaches Raffinationsverfahren ermöglichen, Fettsäuren bzw. Fettsäurealkylester aus natürlichen Quellen unterschiedlichster Qualität so zu vereinheitlichen, dass reproduzierbare Arbeitsergebnisse beispielsweise bei der Sulfonierung der Fettsäurealkylester ermöglicht werden. Insbesondere will die Erfindung auch Ausgangsmaterialien minderer Qualität nachfolgenden Veredelungsstufen zu hochwertigen Materialien zugänglich machen.

Die technische Lösung der erfindungsgemässen Aufgabe geht von der Feststellung aus, dass das angestrebte Ziel erreicht wird, wenn man die Fettsäurealkylester unter den bestimmten, im folgenden angegebenen Bedingungen einer thermischen Vorbehandlung unterwirft oder die freien Fettsäuren dieser Vorbehandlung unterwirft und sie anschliessend mit den entsprechenden Alkoholen verestert.

Gegenstand der Erfindung ist dementsprechend ein Verfahren zur Herstellung von Fettsäurealkylestern mit verbesserter Verarbeitbarkeit. Das erfindungsgemässe Verfahren ist dadurch gekennzeichnet, dass man

a) Alkylester von Fettsäuren pflanzlichen und/oder tierischen Ursprungs in Gegenwart von Veresterungskatalysatoren und/oder Carbonsäureanhydriden in Mengen von 0,01 bis 20 Gew.-%, bezogen auf eingesetzte Fettsäurealkylester einer Temperaturbehandlung oberhalb von 150°C für einen Zeitraum bis zu 60 Minuten unterwirft und gleichzeitig und/oder anschliessend die gereinigten Fettsäurealkylester vom behandelten Gut abtrennt, oder

b) freie Fettsäuren pflanzlichen und/oder tierischen Ursprungs in Gegenwart von Veresterungskatalysatoren in Mengen von 0,01 bis 20 Gew.-%, bezogen auf eingesetzte Fettsäuren, und/oder Carbonsäureanhydriden einer Temperaturbehandlung oberhalb von 150°C für einen Zeitraum bis zu 60 Minuten unterwirft, gleichzeitig und/oder anschliessend die gereinigten Fettsäuren abtrennt und die abgetrennten Fettsäuren in bekannter Weise mit Alkoholen verestert.

Bevorzugt wird diese Temperaturvorbehandlung im Bereich bis 280°C und bevorzugt im Temperaturbereich bon 200 bis 250°C durchgeführt. Temperaturen im Bereich von 200 bis 230°C können besonders zweckmässig sein. Die Verfahrensbedingungen und insbesondere Reaktionstemperaturen und Verweilzeit des zu reinigenden Ausgangsmaterials unter Verfahrensbedingungen werden dabei vorzugsweise in der im folgenden noch zu schildernden Weise aufeinander abgestimmt.

Die Zeitdauer der erfindungsgemässen Vorbehandlung – d.h. die Verweilzeit des zu reinigenden Fettsäurealkylester- bzw. Fettsäure-Ausgangsmaterials unter Verfahrensbedingungen ist im allgemeinen verhältnismässig kurz; sie kann beispielsweise einen Zeitraum bis zu 15 Minuten betragen, wobei in bevorzugten Ausführungsformen der Erfindung allerdings mit beträchtlich kürzeren Verweilzeiten gearbeitet werden kann. Gewünschtenfalls ist es möglich, die erfindungsgemässe thermische Vorbehandlung länger auszudehnen, beispielsweise bis zu 30 Minuten oder auch bis zu einer Stunde, womit jedoch üblicherweise keine wesentlichen verfahrenstechnischen Vorteile verbunden sind.

In der bevorzugten Ausführungsform der Erfindung werden die Bedingungen der erfindungsgemässen Raffinationsstufe und dabei insbesondere die Verfahrenstemperatur und die Verweilzeit des zu behandelnden Gutes derart aufeinander abgestimmt, dass eine Veresterung bzw. Umesterung freier Hydroxylgruppen stattfinden kann, und zwar vorzugsweise ohne dass im übrigen die Struktur der Fettsäurealkylester oder Fettsäuren substantiell verändert wird. Diese erfindungsgemässe Massnahme wird aus dem folgenden verständlich: Das zu reinigende Fettsäurealkylester- oder Fettsäure-Ausgangsmaterial enthält offensichtlich störende Komponenten, die in wechselnden Mengen, bedingt durch Ursprung und individuelle Geschichte des jeweiligen Fettsäure-Materials zugegen sind. Bei diesen störenden Komponenten handelt es sich offenbar um kettenständig hydoxylierte Verbindungen, die gegebenenfalls nur in Spurenmengen vorliegen, bei der späteren Verarbeitung der Fettsäurealkylester in einem der geschilderten Verfahren sich aber überproportional störend bemerkbar machen. Das erfindungsgemässe Verfahren will durch die Temperaturvorbehandlung unter den angegebenen Bedingungen die Möglichkeit schaffen, ein standartisiertes gereinigtes Fettsäurealkylester-Produkt zu gewinnen, das in den nachfolgenden Verarbeitungsstufen die geschilderten Nachteile nicht mehr aufweist. Tatsächlich ermöglicht es die erfindungsgemässe Vorbehandlung, auch kleinste Mengen störender Begleitstoffe auf reaktivem Wege von den kettenständig nicht hydroxylierten Fettsäurealkylestern bzw. Fettsäuren abzutrennen. Durch Veresterung bzw. Umesterung der freie Hydroxylgruppen enthaltenden Komponenten mit den im Überschuss vorliegenden Ester- bzw. Carboxylgruppierungen des Einsatzgutes und/oder durch Umsetzung mit den zugesetzten Carbonsäureanhydriden findet bezüglich dieser störenden Beimischungen eine solche Siedepunktverschiebung statt, dass die destillative Abtrennung der gereinigten Hauptmasse des Einsatzmaterials von den gebildeten Hochsiedern möglich wird. Fettsäurealkylester bzw. Fettsäuren natürlichen Ursprungs stellen üblicherweise Mischungen wechselnder Mengen von gesättigten und ungesättigten Verbindungen dar, so wie sie aus den jeweiligen Fetten bzw. Ölen natürlichen Ursprungs gewonnen worden sind. Durch ihre individuelle Vorgeschichte bei Gewinnung und Lagerung, durch Abmischungen bzw. Verschnitte und weitere Einflüsse dieser Art muss die Praxis stets mit Ausgangsmaterialien stark wechselnder Qualifikationen rechnen. Durch die erfindungsgemässe Vorbehandlung gelingt die Umwandlung beliebig unterschiedlicher Ausgangsmaterialien in stets gleicher Weise zu einem Produkt, das in einer der geschilderten nachfolgenden Behandlungsstufen leichter verarbeitet werden kann und/oder zu verbesserten Endprodukten führt.

Die erfindungsgemässe reaktive Reinigung kann durch Arbeiten unter verminderten Drucken begünstigt werden. Eine solche Druckminderung dürfte die Verschiebung des Gleichgewichts bei der Umesterungsreaktion zwischen kettenständigen Hydroxylgruppen und der endständigen Estergruppierung unter Abspaltung und Entfernung des monofunktionellen Alkohols aus dem eingesetzten Fettsäurealkylester beschleunigen. In einer bevorzugten Ausführungsform wird dabei mit derart vermindertem Druck gearbeitet, dass gleichzeitig mit oder unmittelbar anschliessend an die erfindungsgemässe Raffinationsbehandlung die Destillation der von störenden Komponenten befreiten Fettsäurealkylester bzw. Fettsäuren ermöglicht wird. So kann beispielsweise das Arbeiten im Druckbereich von 0,067 bis 13,3 mbar (0,05– 10 Torr), bevorzugt im Bereich von 0,13 bis 6,65 mbar (0,1 bis 5 Torr) besonders zweckmässig sein. Es gelingt auf diese Weise, die erfindungsgemässe Temperaturvorbehandlung zur reaktiven Beseitigung der störenden Komponenten und die destillative Abtrennung des gereinigten Gutes in einer vorzugsweise kontinuierlich betriebenen Verfahrensstufe zusammenzufassen. Die reaktive Umwandlung der störenden Begleitkomponenten zu Hochsiedern gelingt dabei in erstaunlich kurzen Zeiträumen, die im Bereich von Sekunden oder gar nur Bruchteilen einer Sekunde liegen können. So ist es erfindungsgemäss möglich, Veresterungskatalysatoren und/oder Carbonsäureanhydride dem zu reinigenden Einsatzmaterial zuzusetzen und dann das Gemisch der Destillation zu unterwerfen, wobei darauf zu achten ist, dass die Sumpftemperatur des zu destillierenden Gutes oberhalb 150 °C und vorzugsweise innerhalb des erfindungsgemäss besonders geeigneten Temperaturbereiches liegt. Thermische Vorbehandlung und Abtrennung der unerwünschten Produktanteile sind damit praktisch in einem Verfahrensschritt zusammengefasst. Eine andere für die technische Durchführung besonders einfache Modifikation sieht vor, Veresterungskatalysatoren und/oder Carbonsäureanhydride – beispielsweise in einem Hochsieder gelöst – im Temperaturbereich der erfindungsgemässen Vorbehandlung und unter vermindertem Druck vorzulegen. Das zu reinigende Ausgangsmaterial wird dieser Reaktionszone absatzweise oder vorzugsweise kontinuierlich zugeführt. Dabei sind Druck- und Temperaturbedingungen so aufeinander abgestimmt, dass das Abdestillieren der Hauptmasse des Fettsäurealkylester- bzw. Fettsäure-Materials gewährleistet ist. Trotz des sehr raschen Verdampfens des in die Reaktionszone eingebrachten Ausgangsmaterials findet die reaktive Umwandlung der eingetragenen störenden Komponenten in solchem Ausmass statt, dass das anfallende Destillat die angestrebte verbesserte Verarbeitbarkeit zeigt.

Insbesondere in diesen zuletzt genannten Ausführungsformen der gleichzeitigen reaktiven Reinigung und Destillation kann beispielsweise mit Verweilzeiten im Bereich von 0,1 bis 3 Minuten gearbeitet werden, wobei zweckmässigerweise die Temperaturen im Bereich von 200 bis 250 °C liegen.

Als reaktive Hilfsstoffe werden im erfindungsgemässen Verfahren Veresterungskatalysatoren und/oder Carbonsäureanhydride eingesetzt. Es können dabei beliebige Veresterungskatalysatoren aus dem umfangreichen einschlägigen Stand der Technik Verwendung finden. Bevorzugt wird allerdings mit basischen, neutralen oder äusserstenfalls schwach sauren Katalysatoren bzw. Katalysatorsystemen gearbeitet. Besonders bevorzugt ist das Arbeiten mit Katalysatoren, die im zu reinigenden Einsatzmaterial und/oder in Hochsiedern löslich sind, die sich bei dem erfindungsgemässen Verfahren bilden und/oder bei der erfindungsgemässen Reinigung im zuvor geschilderten Sinne eingesetzt werden. Die Verwendung von flüssigen löslichen Katalysatoren bzw. Katalysatorsystemen kann besonders zweckmässig sein.

Aus dem umfangreichen Stand der Technik zu Veresterungskatalysatoren sei hier lediglich beispielhaft zitiert die Zusammenstellung in J. Am. Oil Chem. Soc. 55, 796 und folgende (1978), insbesondere die Zusammenstellung aus Tabelle I auf Seite 797. Aufgezählt sind hier Metallsalze wie Acetate, Carbonate, Chloride, Nitrate, Oxide von Zinn, Zink, Eisen, Cobalt und Blei, Alkalihydroxide wie NaOH, KOH, LiOH, Metallseifen wie die Stearate von Alkalimetallen, Zink, Aluminium und Titan, Alkalimetalle bzw. ihre Legierungen, Metallalkylate und Metallhydride. Geeignet können insbesondere sein Metallseifen gesättigter und/oder ungesättigter mono- bzw. polyfunktioneller Carbonsäuren der Kohlenstoffkettenlänge $C_2$–$C_{36}$ folgender Metalle: K, Na, Li, Al, B, Zn, Sn, Ca, Mg, Ti und V, Metallalkoholate gesättigter und ungesättigter mono- bzw. polyfunktioneller linearer oder verzweigter Alkohole mit der Kohlenstoffkettenlänge $C_1$–$C_{36}$ mit folgenden Metallen als Kation: Li, Na, K, Mg, Ca, B, Al, Zn, Sn und Ti, als Metallhydride Wasserstoffverbindungen der Elemente oder deren Mischungen von Li, Na, Mg, Ca, B, Al und Sn und als Metallalkyle Kohlenstoffverbindungen der Elemente oder deren Mischungen von Li, Na, Mg, Ca, B, Al, Sn und Ti.

Eine besonders geeignete Klasse von Katalysatoren bzw. reaktiven Hilfsstoffen leitet sich von löslichen organischen Verbindungen der Borsäure ab. In Betracht kommen hier insbesondere Borsäureester, beispielsweise Borsäurealkylester, von denen solche mit vergleichsweise höheren Alkoholen ($C_{10}$–$C_{22}$, insbesondere $C_{12}$–$C_{18}$-Alkohole) besonders bevorzugt sein können. Geeignet sind insbesondere aber auch Ester der Borsäure mit Teilestern polyfunktioneller Alkohole, beispielsweise entsprechende Ester mit Mono- bzw. Diglyceriden. Diese Teilester polyfunktioneller Alkohole können sich ebenfall von höheren Alkoholen, insbesondere solchen des C-Zahlbereiches von 10 bis 22 ableiten. Unter Verfahrenstemperatur flüssig und/oder lösliche Borsäureester können eine bevorzugte Katalysatorklasse sein. Weitere Beispiele für geeignete Borsäureverbindungen sind Borsäureanhydrid, Natriumborat und Natriumboranat.

Neben oder anstelle von Veresterungskatalysatoren können als reaktive Hilfsstoffe bei der erfindungsgemässen Temperaturbehandlung Carbonsäureanhydride mitverwendet werden. Es ist allerdings bevorzugt, in dieser Ausführungsform Veresterungskatalysatoren mitzuverwenden, die die Reaktion der Carbonsäureanhydridgruppierung mit Hydroxylgruppen begünstigen. Die Carbonsäureanhydride können sich grundsätzlich von Monocarbonsäuren oder Polycarbonsäuren – hier insbesondere von Dicarbonsäuren – ableiten. In einer Ausführungsform der Erfindung kann es bevorzugt sein, vergleichsweise hochsiedende Carbonsäureanhydride einzusetzen, deren Siedepunkt insbesondere höher als der Siedepunkt der zu raffinierenden Fettsäurealkylester bzw. Fettsäuren liegt. So können beispielsweise oberhalb 300 °C/1,33 mbar (1 Torr) siedende Carbonsäureanhydride ein geeignetes Ausgangsmaterial für das Verfahren der Erfindung sein. Solche Carbonsäureanhydride leiten sich beispielsweise von $C_8$–$C_{28}$-Monocarbonsäuren, insbesondere von Monocarbonsäuren mit 10–22 C-Atomen ab. Das Arbeiten mit derart hochsiedenden Carbonsäureanhydriden erleichtert das erfindungsgemässe Verfahren insoweit, als evtl. vorliegende Überschüsse dieses reaktiven Hilfsstoffs durch eine anschliessende Destillation leicht von den gereinigten Fettsäurealkylestern bzw. Fettsäuren abgetrennt werden können.

Unter den Begriff der Carbonsäureanhydride fallen erfindungsgemäss auch gemischte Säureanhydride aus Carbonsäuren und anorganischen Säuren, insbesondere Borsäure. Wie angegeben kann es für die Durchführung des erfindungsgemässen Verfahrens im kontinuierlichen Betrieb wünschenswert sein, dass die als Hilfsstoffe eingesetzten Reaktionskomponenten fliessfähig sind. Destillationsrückstände aus der bekannten Behandlung von Fettsäuren, beispielsweise solchen natürlichen Ursprungs, mit Borsäureverbindungen, die zu einem nicht unbeträchtlichen Anteil Fettsäureanhydride, gemischte Borsäure-Fettsäureanhydride und/oder Borsäure enthalten, sind ein für das erfindungsgemässe Reinigungsverfahren geeigneter Zusatzstoff. Diese fliessfähigen Destillationsrückstände können somit einer wichtigen weiteren Verarbeitung zugeführt werden.

Die genannten reaktiven Hilfsstoffe (Veresterungskatalysatoren und/oder Carbonsäureanhydride) werden dem zu reinigenden Ausgangsmaterial üblicherweise in nur geringen Mengen zugesetzt, wenn auch grössere Mengen grundsätzlich nicht schädlich sind. Geeignet sind beispielsweise Mengen von 0,01 bis 20 Gew.-%, bevorzugt Mengen im Bereich von 0,1 bis 10 Gew.-% des reaktiven Hilfsstoffs bzw. Hilfsstoffgemisches – jeweils bezogen auf das Fettsäurealkylester- bzw. Fettsäure-Ausgangsmaterial.

Im allgemeinen werden Mengen nicht über 5 Gew.-%, vorzugsweise nicht über 3 Gew.-% des reaktiven Hilfsstoffs zum Einsatz kommen. Der besonders bevorzugte Bereich für die einzusetzende Menge liegt zwischen 0,05 und 1,0 Gew.-%. Alle diese Gewichtsprozentangaben beziehen sich auf das zu behandelnde Fettsäurealkylester- bzw. Fettsäure-Einsatzmaterial. Werden Carbonsäureanhydride zusammen mit Veresterungskatalysatoren eingesetzt, so können die Katalysatoren in den üblichen geringen Mengen zur Verwendung kommen, beispielsweise beträgt ihre Menge 0,001 bis 10 Gew.-%, insbesondere 0,01 bis 5 Gew.-% – bezogen auf das Carbonsäureanhydrid. Wird im eingangs geschilderten kontinuierlichen Verfahren derart gearbeitet, dass zu reinigendes Einsatzmaterial einer Reaktionszone zugegeben wird, die Veresterungskatalysatoren und/oder Carbonsäureanhydride enthält, wobei gleichzeitig gereinigtes Fettsäurealkylester- bzw. Fettsäure-Material destillativ aus dieser Reaktionszone abgezogen wird, so ist lediglich darauf zu achten, dass die vorgelegte Menge an Umesterungskatalysator und/oder Carbonsäureanhydrid ausreicht, die gewünschte reaktive Beseitigung der störenden Komponenten in der kurzen zur Reaktion zur Verfügung stehenden Zeitspanne zu bewirken.

Die erfindungsgemässe Reinigung der Fettsäurealkylester ergibt weder eine Farbverbesserung noch eine wesentliche Änderung der Kennzahlen (Hydroxylzahl, Jodzahl, Verseifungszahl und/oder Säurezahl). Der Effekt der erfindungsgemässen Vorbehandlung zeigt sich nicht am Fettsäurealkylester selber, sondern erst bei seiner weiteren Verarbeitung, z.B. nach der Sulfonierung eines derart vorbehandelten Ausgangsmaterials. Hier drückt er sich beispielsweise in der besseren Bleichbarkeit des Sulfonierungsprduktes aus. Gebleichte Produkte mit Klett-Farbzahlen unter 60 sind problemlos herzustellen.

Die erfindungsgemässe Vorbehandlung kann an hydrierend gehärteten, gewünschtenfalls aber auch an noch ungehärteten Fettsäurealkylestern- bzw. Fettsäurefraktionen vorgenommen werden. Wird ungehärtetes Material behandelt, dann empfiehlt es sich, die gewonnene Produktionsfraktion baldmöglichst der Härtung zuzuführen, um unerwünschte oxidative Einflüsse über die im Produkt vorliegenden Doppelbindungen durch Alterung auszuschliessen.

Eine konventionelle Abtrennung störender Komponenten – vermutlich der durch oxidative Alterung gebildeten Anteile an kettenständig hydroxylierten Fettsäuremethylestern – von den unproblematischen Anteilen des Ausgangsmaterials ist praktisch nicht möglich. Eine Destillation führt nicht zum Ziel. Durch die Lehre der Erfindung, das beliebig zusammengesetzte Ausgangsmaterial mit den reaktiven Hilfsstoffen der thermischen Vorbehandlung zu unterwerfen und insbesondere über diesem Material zu destillieren, beseitigt die bestehenden Schwierigkeiten und liefert zuverlässig ein für die anschliessende Sulfonierung und Bleiche oder Verseifung brauchbares Ausgangsmaterial.

Für die nachfolgende Sulfonierung und Bleiche gelten die Angaben des Standes der Technik. Einzelheiten zur Durchführung der sauren Bleiche oder mehrstufigen Kombinationsbleiche finden sich beispielsweise in den DE-PS 1 179 931, 1 234 709 und der DE-OS 1 443 995. Die vorgängige Sulfonierung wird üblicherweise bei Temperaturen von 70 bis 130 °C in einem Fallfilmreaktor mit einem Gemisch von gasförmigem Schwefeltrioxid und Inertgas während eines Zeitraums von 10 bis 20 Minuten bei Sulfonierungsgraden von mehr als 90, insbesondere mehr als 92% und in der Regel mehr als 94% durchgeführt. Sulfonierungsgrade von 95% und darüber sind besonders bevorzugt. Gleichwohl können unter Mitverwendung der erfindungsgemässen Reinigungsbehandlung letztlich Sulfonierungsprodukte mit Klett-Farbzahlen von 50 oder darunter zuverlässig hergestellt werden.

Als Fettsäurealkylester-Ausgangsmaterial eignen sich insbesondere die entsprechenden niederen Alkylester mit vorzugsweise 1 bis 5 Kohlenstoffatomen im Alkoholrest. Besondere Bedeutung kommt den Fettsäuremethylestern zu, die aus pflanzlichen und/oder tierischen Fetten durch Umesterung oder durch Verseifung mit nachfolgender Veresterung gewonnen worden sind.

Die nachfolgenden Beispiele schildern die erfindungsgemässe Vorbehandlung und ihre Ergebnisse anhand von Vergleichsversuchen, die ausserhalb der Erfindung liegen.

Der Reinigungseffekt des Arbeitens nach der Erfindung wird an der Bleichbarkeit der nach der Sulfonierung erhaltenen Estersulfonatpasten gemessen.

Die nachfolgend beschriebene Methode der Sulfonierung und Bleichung gilt für alle Beispiele:

Jeweils 576 g Talgfettsäuremethylester wurden in einem auf 80 °C erwärmten Standzylinder durch 65-minütiges Einblasen eines 5 Vol.-%igen $SO_3$/Luft-Gemisches, das eine Gesamtmenge von 208 g ($\hat{=}$ 2,6 Mol) $SO_3$ enthielt, und einer anschliessenden Nachreaktionszeit von 15 Minuten sulfoniert. Die erhaltene Rohsulfonsäure wurde durch gleichzeitiges Zusammenführen von Rohsulfonsäure mit Natronlauge im pH-Bereich von 6,5 bis 8 zu einer ca. 25 Gew.-% Sulfonierungsprodukt enthaltenden wässrigen Paste neutralisiert, die anschliessend mit 15,4 Gew.-% einer 13-%igen wässrigen NaOCl-Lösung – bezogen auf Sulfonierungsprodukt – bei 60 °C gebleicht wurde. Eine in Bezug auf Sulfonierungsprodukt 5-%ige wässrige Lösung, eingestellt auf pH 7, im Klettphotometer (Modell 800–3 der Fa. Klett-Summerson) mit Blaufilter (420 nm) in einer Klett-Rundglas-Küvette eine Klettfarbzahl, wie sie jeweils in den einzelnen Beispielen angegeben ist. Der Sulfiergrad der erhaltenen Pasten lag zwischen 95 und 97%.

Vergleichsversuch 1

Als Ausgangsmaterial diente ein gehärteter (hydrierter) Talgfettsäuremethylester, gewonnen durch Spaltung von Talg, Auswaschen des Glycerins, Veresterung der Talgfettsäure mit Methanol, anschliessende Härtung (Hydrierung der vorhandenen C–C-Doppelbindungen) und Destillation. Dieser gehärtete Talgfettsäuremethylester mit den Kennzahlen: Jodzahl 0,3; Hydroxylzahl 2,0; Säurezahl 0,6; Verseifungszahl 194,4 wurde wie oben beschrieben sulfoniert, neutralisiert und gebleicht. Nach einer Bleichzeit von 30 Minuten betrug die Klettzahl der Estersulfonatpaste 255.

Beispiel 1

Der im Vergleichsversuch 1 verwendete gehärtete Talgfettsäuremethylester wurde vor der Sulfonierung über 0,5 Gewichtsprozent Lithiumaluminiumhydrid, bezogen auf Fettsäuremethylester, bis zu einer Sumpftemperatur von 230 °C/0,1 mbar destilliert. Der Destillationsrückstand abzüglich des Lithiumaluminiumhydrids betrug 8,5 Gewichtsprozent. Der auf diese Weise gereinigte gehärtete Talgfettsäuremethylester hatte die Kennzahlen: Iodzahl 0,1; Hydroxylzahl 0; Säurezahl 0,2; Verseifungszahl 194,3; er wurde unter den oben angegebenen Bedingungen sulfoniert, neutralisiert und gebleicht. Nach einer Bleichzeit von 30 Minuten betrug die Klettzahl der Estersulfonatpaste 42.

Beispiel 2

Der im Vergleichsversuch 1 verwendete gehärtete Talgfettsäuremethylester wurde vor der Sulfonierung in Gegenwart von 0,5 Gewichtsprozent Aluminiumchlorid, bezogen auf Fettsäuremethylester, bis zu einer Sumpftemperatur von 230 °C/0,1 mbar destilliert. Der Destillationsrückstand abzüglich des Aluminiumchlorids betrug 2,8 Gewichtsprozent. Der gereinigte gehärtete Talgfettsäuremethylester hatte die Kennzahlen: Iodzahl 0,2, Hydroxylzahl 0; Säurezahl 0,2; Verseifungszahl 193,8; er wurde wie oben beschrieben sulfoniert, neutralisiert und gebleicht. Nach einer Bleichzeit von 30 Minuten betrug die Klettzahl der Estersulfonatpaste 50.

Beispiel 3

Der im Vergleichsversuch 1 verwendete gehärtete Talgfettsäuremethylester wurde vor der Sulfonierung mit einem Zusatz von 0,5 Gewichtsprozent Natriumaluminiumhydridotrimethylat NaAlH(OCH$_3$)$_3$ destilliert; der Destillationsrückstand

abzüglich des Katalysators betrug 2,4 Gewichtsprozent. Der gereinigte gehärtete Talgfettsäuremethylester hatte die Kennzahlen: Iodzahl 0,2; Hydroxylzahl 0; Säurezahl 0,3; Verseifungszahl 194,2; er wurde wie oben beschrieben sulfoniert, neutralisiert und gebleicht. Nach der Bleichzeit von 30 Minuten betrug die Klettzahl der Estersulfonatpaste 45.

Vergleichsversuch 2

Als Ausgangsmaterial diente ein gehärteter (hydrierter) Talgfettsäuremethylester, der durch Spaltung von Talg, Auswaschen des Glycerins, Veresterung der Talgfettsäure mit Methanol, Hydrierung der vorhandenen C–C-Doppelbindungen und Destillation erhalten worden war. Dieser gehärtete Talgfettsäuremethylester mit den Kennzahlen: Iodzahl 0,2; Hydroxylzahl 1,8; Säurezahl 0,2; Verseifungszahl 194,4 wurde wie oben beschrieben sulfoniert, neutralisiert und gebleicht. Nach einer Bleichzeit von 2 Stunden betrug die Klettzahl 200.

Beispiel 4

Der im Vergleichsversuch 2 als Ausgangsmaterial verwendete gehärtete Talgfettsäuremethylester wurde vor der Sulfonierung in Gegenwart von 5 Gewichtsprozent Borsäure bis zu einer Sumpftemperatur von 230°C/1 mbar destilliert. Der Destillationsrückstand abzüglich der vorhandenen Borsäure betrug 0,8 Gewichtsprozent. Dieser Talgfettsäuremethylester wurde in der angegebenen Weise sulfoniert, neutralisiert und gebleicht. Nach einer Bleichzeit von 2 Stunden betrug die Klettzahl 33.

Vergleichsversuch 3

Ein gehärteter Talgfettsäuremethylester, gewonnen wie im Vergleichsversuch 1, mit den Kennzahlen: Iodzahl 0,3; Hydroxylzahl 2,0; Säurezahl 0,6; Verseifungszahl 194,4, wurde wie bereits beschrieben sulfoniert, neutralisiert und gebleicht. Nach einer Bleichzeit von 4 Stunden betrug die Klettzahl der Estersulfonatpaste 200.

Beispiel 5

Der im Vergleichsversuch 3 verwendete gehärtete Talgfettsäuremethylester wurde vor der Sulfonierung in Gegenwart von 2 Gewichtsprozent Aluminiumstearat bis zu einer Sumpftemperatur von 230°C/1 mbar destilliert. Der Destillationsrückstand abzüglich des vorhandenen Aluminiumstearats betrug 3,5 Gewichtsprozent. Der gereinigte gehärtete Talgfettsäuremethylester mit Iodzahl 0,3; Hydroxylzahl 0; Säurezahl 0,8; Verseifungszahl 194,1 wurde wie oben angegeben sulfoniert, neutralisiert und gebleicht. Nach einer Bleichzeit von 4 Stunden betrug die Klettzahl der Estersulfonatpaste 50.

Beispiel 6

Der im Vergleichsversuch 3 verwendete gehärtete Talgfettsäuremethlyester wurde vor der Sulfonierung unter Zusatz von 1 Gewichtsprozent Eisen(III)-chlorid bis zu einer Sumpftemperatur von 230°C/1 mbar destilliert. Der Destillationsrückstand, abzüglich des vorhandenen Eisen(III)-chlorids, betrug 4,5 Gewichtsprozent. Der auf diese Weise gereinigte gehärtete Talgfettsäuremethylester mit Iodzahl 0,6; Hydroxylzahl <1; Säurezahl 0,2; Verseifungszahl 194,0 wurde wie zuvor beschrieben sulfoniert, neutralisiert und gebleicht. Nach einer Bleichzeit von 4 Stunden betrug die Klettzahl der Estersulfonatpaste 80.

Vergleichsversuch 4

Als Ausgangsmaterial diente ein gehärteter Talgfettsäuremethylester, gewonnen durch Umesterung von Talg mit Methanol mit anschliessender Destillation und Härtung (Hydrierung der vorhandenen C–C-Doppelbindungen) mit den Kennzahlen: Iodzahl 0,55; Hydroxylzahl <1; Säurezahl 0,4; Verseifungszahl 194,0. Dieser Ester wurde sulfoniert, neutralisiert und gebleicht. Nach einer Bleichzeit von 30 Minuten betrug die Klettzahl der erhaltenen Estersulfonatpaste 260.

Vergleichsversuch 5

Der im Vergleichsversuch 4 verwendete Talgfettsäuremethylester mit der Iodzahl 0,55 wurde auf eine Iodzahl von 0,1 gehärtet. Dieser Ester wurde wie beschrieben sulfoniert, neutralisiert und gebleicht. Nach einer Bleichzeit von 30 betrug die Klettzahl der Estersulfonatpaste 180.

Beispiel 7

Der im Vergleichsversuch 5 verwendete nachgehärtete Talgfettsäuremethylester wurde vor der Sulfonierung unter Zusatz von 0,5 Gewichtsprozent Natriumborhydrid bis zu einer Sumpftemperatur von 230°C/1 mbar destilliert. Der Destillationsrückstand betrug 2,8 Gewichtsprozent. Der auf diese Weise gereinigte Talgfettsäuremethylester wurde wie oben beschrieben sulfoniert, neutralisiert und gebleicht. Nach einer Bleichzeit von 30 Minuten betrug die Klettzahl der erhaltenen Estersulfonatpaste 40.

Beispiel 8

Zur Herstellung des Katalysators wurden 379 g Talgfettsäure und 7,5 g Borsäure 3 Stungen lang bei 200°C gerührt. Anschliessend wurde das erhaltene Gemisch bis zu einer Sumpftemperatur von 280°C/0,1 mbar destilliert. Es wurden 82 g Rückstand mit einem Schmelzpunkt von ca. 60°C erhalten.

Das im Vergleichsversuch 5 verwendete Ausgangsmaterial wurde vor der Sulfonierung in Gegenwart von 1,5 Gewichtsprozent des Katalysators, bezogen auf eingesetzten Talgfettsäuremethylester, bis zu einer Sumpftemperatur von 230°C/0,1 mbar destilliert. Der Destillationsrückstand betrug 3 Gewichtsprozent. Der destillierte gehärtete Talgfettsäuremethylester wurde in der beschriebenen Weise sulfoniert, neutralisiert und gebleicht. Nach einer Bleichzeit von 30 Minuten betrug die Klettzahl der erhaltenen Estersulfonatpaste 27.

Vergleichsversuch 6

Als Ausgangsmaterial diente ein gehärteter Fettsäuremethylester, gewonnen durch Umesterung von Talg mit Methanol mit anschliessender Destillation und Härtung. Dieses Material wurde einer zweiten Destillation unterworfen. Der erhaltene gehärtete Fettsäuremethylester war frei von Glyceriden und hatte folgende Kennzahlen: lodzahl 0,25; Hydroxylzahl 1,0; Säurezahl 0,2; Verseifungszahl 196,4. Dieser Ester wurde auf die beschriebene Weise sulfoniert, neutralisiert und gebleicht. Nach einer Bleichzeit von 2 Stunden betrug die Klettzahl der erhaltenen Estersulfonatpaste 95.

Beispiel 9

Zur Herstellung des Katalysators wurden 500 g Talgfettsäure und 2,5 g Borsäure eine Stunde lang bei 200 °C/133 mbar geührt und das Gemisch anschliessend bis zu einer Sumpftemperatur von 280 °C/0,1 mbar destilliert. Der Rückstand betrug 120 g.

Das im Vergleichsversuch 6 verwendete Ausgangsmaterial wurde vor der Sulfonierung unter Zusatz von 1,3 Gewichtsprozent des zuvor hergestellten Katalysators, bezogen auf Fettsäuremethylester, bis zu einer Sumpftemperatur von 230 °C/0,1 mbar destilliert. Der Destillationsrückstand betrug 1,6 Gewichtsprozent. Der gereinigte Talgfettsäuremethylester wurde wie oben beschrieben sulfoniert, neutralisiert und gebleicht. Nach einer Bleichzeit von 2 Stunden betrug die Klettzahl der erhaltenen Estersulfonatpaste 30.

Vergleichsversuch 7

Als Ausgangsmaterial diente ein Talgfettsäuremethylester, gewonnen durch Umesterung von Talg mit Methanol mit anschliessender Destillation und Härtung. Das gehärtete Material wurde einer zweiten Destillation unterworfen. Der erhaltene Fettsäuremethylester war frei von Glyceriden und hatte die Kennzahlen: lodzahl 0,2; Hydroxylzahl 0,8; Säurezahl 0,6; Verseifungszahl 196,6. Dieser gehärtete Talgfettsäuremethylester wurde wie beschrieben sulfoniert, neutralisiert und gebleicht. Nach einer Bleichzeit von 30 Minuten betrug die Klettzahl der erhaltenen Estersulfonatpaste 135.

Beispiel 10

Zur Herstellung des Katalysators wurden 320 g eines Gemisches aus 45 Gewichtsprozent Glycerinmonostearat, 41 Gewichtsprozent Glycerindistearat und 14 Gewichtsprozent Glycerintristearat zusammen mit 62 g Borsäure bei 160 °C/66,5 mbar 3 Stunden lang gerührt. Dabei löste sich die Borsäure auf. Das Reaktionsprodukt hatte einen Schmelzpunkt von ca. 50 °C.

Das im Vergleichsversuch 7 verwendete Ausgangsmaterial wurde vor der Sulfonierung in Gegenwart von 0,9 Gewichtsprozent des zuvor hergestellten Katalysators, bezogen auf eingesetzten Fettsäureester, bis zu einer Sumpftemperatur von 230 °C/0,1 mbar destilliert. Der Destillationsrückstand betrug 1,7 Gewichtsprozent. Der destillierte gehärtete Talgfettsäuremethylester wurde in der beschriebenen Weise sulfoniert, neutralisiert und gebleicht. Nach einer Bleichzeit von 30 Minuten betrug die Klettzahl der erhaltenen Estersulfonatpaste 46.

Beispiel 11

Das im Vergleichsversuch 6 verwendete Ausgangsmaterial wurde vor der Sulfonierung in Gegenwart von 1,0 Gewichtsprozent Zinkstearat bis zu einer Sumpftemperatur von 230 °C/0,1 mbar destilliert. Der Destillationsrückstand betrug 2,0 Gewichtsprozent. Der destillierte gehärtete Talgfettsäuremethylester wurde wie beschrieben sulfoniert, neutralisiert und gebleicht. Nach einer Bleichzeit von 2 Stunden betrug die Klettzahl der erhaltenen Estersulfonatpaste 47.

Beispiel 12

Das im Vergleichsversuch 7 verwendete Ausgangsmaterial wurde vor der Sulfonierung mit einem Zusatz von 0,5 Gewichtsprozent Natriummethylat, bezogen auf den Fettsäureester, bis zu einer Sumpftemperatur von 230 °C/0,1 mbar destilliert. Der Destillationsrückstand betrug 6,3 Gewichtsprozent. Der gereinigte Talgfettsäuremethylester wurde sulfoniert, neutralisiert und gebleicht. Nach einer Bleichzeit von 30 Minuten betrug die Klettzahl der Estersulfonatpaste 40.

Beispiel 13

Zur Herstellung des Katalysators wurden 379 g gehärtete Talgfettsäure (lodzahl 0,3) und 7,5 g Borsäure 3 Stunden lang bei 200 °C gerührt und das Gemisch anschliessend bis zu einer Sumpftemperatur von 280 °C/0,1 mbar destilliert. Es wurden 82 g Rückstand mit einem Schmelzpunkt von ca. 60 °C erhalten.

In dem im Vergleichsversuch 5 verwendeten Ausgangsmaterial wurden 1,5 Gewichtsprozent des Katalysators, bezogen auf eingesetzten Fettsäuremethylester, aufgelöst. Dieses Gemisch wurde in eine auf 230 °C erhitzte Destillationsblase bei 0,1 mbar kontinuierlich eingetropft, wobei der Zulauf so geregelt wurde, dass dieser der übergehenden Destillatmenge entsprach. Der Destillationsrückstand betrug 2 Gewichtsprozent. Der auf diese Weise destillierte Talgfettsäuremethylester wurde wie zuvor beschrieben sulfoniert, neutralisiert und gebleicht. Nach einer Bleichzeit von 30 Minuten betrug die Klettzahl der erhaltenen Estersulfonatpaste 50.

Beispiel 14

Als Ausgangsmaterial diente eine ungehärtete Talgfettsäure, gewonnen durch die Spaltung von Talg und Auswaschen des Glycerins. Diese Talgfettsäure wurde in Gegenwart von 1 Gewichtsprozent Natriumborhydrid, bezogen auf eingesetzte Fettsäure, destilliert. Die destillierte Talgfettsäure wurde mit Methanol im Gewichtsverhältnis 1:1,1 in einem Autoklaven 2 Stunden lang auf 200 °C erhitzt. Danach wurde nicht umgesetztes Methanol abdestilliert. Der Rückstand wurde wiederum mit 1,1 Gewichtsteilen Methanol auf 1 Gewichtsteil eingesetzter Talgfettsäure versetzt und erneut 2 Stunden lang im Autoklaven auf 200°C erhitzt. Diese Operation wurde nach dem Abdestillieren des nicht umgesetzten Methanols aus dem Reaktionsgemisch noch einmal wiederholt. Der dann nach dem Abdestillieren des Methanols zurückbleibende Talgfettsäuremethylester wurde unter Zusatz von 0,3 Gewichtsprozent Raney-Nickel, bezogen auf eingesetzten Fettsäureester, in einem Autoklaven bei 200–220°C unter einem Wasserstoffdruck von 20 bar im Verlauf von 2 Stunden hydriert. Der nach dem Abtrennen des Katalysators erhaltene gehärtete Talgfettsäuremethylester hatte die Kennzahlen: Iodzahl 0,1; Hydroxylzahl 0; Säurezahl 5,9; Verseifungszahl 195,4. Dieser Ester wurde in der beschriebenen Weise sulfoniert, neutralisiert und gebleicht. Nach einer Bleichzeit von 30 Minuten betrug die Klettzahl der erhaltenen Estersulfonatpaste 42.

Beispiel 15

Eine ungehärtete Talgfettsäure, gewonnen durch Spaltung von Talg und Auswaschen des Glycerins, wurde unter den in Beispiel 14 angegebenen Bedingungen hydriert. Die gehärtete Talgfettsäure wurde in Gegenwart von 1 Gewichtsprozent Natriumborhydrid destilliert und danach unter den in Beispiel 14 angegebenen Bedingungen mit Methanol verestert. Der erhaltene gehärtete Talgfettsäuremethylester mit den Kennzahlen: Iodzahl 0,5; Hydroxylzahl 0; Säurezahl 5,5; Verseifungszahl 198 wurde wie zuvor beschrieben sulfoniert und gebleicht. Nach einer Bleichzeit von 2 Stunden betrug die Klettzahl der erhaltenen Estersulfonatpaste 44.

Beispiel 16

Eine ungehärtete Talgfettsäure, gewonnen durch Spaltung von Talg und Auswaschen des Glycerins wurde unter Zusatz von 1 Gewichtsprozent des Katalysators aus Beispiel 10, bezogen auf eingesetzte Fettsäure, destilliert. Die gereinigte Talgfettsäure wurde unter den in Beispiel 14 angegebenen Bedingungen mit Methanol verestert. Der gewonnene Methylester wurde unter den in Beispiel 14 angegebenen Bedingungen hydriert. Der gehärtete Talgfettsäuremethylester hatte die Kennzahlen: Iodzahl 0; Hydroxylzahl 0; Säurezahl 5,4; Verseifungszahl 195,4; er wurde in der angegebenen Weise sulfoniert, neutralisiert und gebleicht. Nach einer Bleichzeit von 30 Minuten betrug die Klettzahl der erhaltenen Estersulfonatpaste 50.

Beispiel 17

Eine ungehärtete Talgfettsäure, gewonnen durch Spaltung von Talg und Auswaschen des Glycerins, wurde unter den in Beispiel 14 angegebenen Bedingungen hydriert. Die erhaltene gehärtete Talgfettsäure wurde unter Zusatz von 5 Gewichtsprozent des Katalysators aus Beispiel 10, bezogen auf eingesetzte Fettsäure, destilliert und anschliessend unter den in Beispiel 14 angegebenen Bedingungen mit Methanol verestert. Der erhaltene gehärtete Talgfettsäuremethylester

hatte die Kennzahlen: Iodzahl 0,4; Hydroxylzahl 0; Säurezahl 2,7; Verseifungszahl 198; er wurde wie zuvor beschrieben sulfoniert, neutralisiert und gebleicht. Nach einer Bleichzeit von 2 Stunden betrug die Klettzahl der erhaltenen Estersulfonatpaste 38.

**Patentansprüche**

1. Verfahren zur Herstellung von Fettsäurealkylestern mit verbesserter Verarbeitbarkeit, dadurch gekennzeichnet, dass man

a) Alkylester von Fettsäuren pflanzlichen und/oder tierischen Ursprungs in Gegenwart von Veresterungskatalysatoren und/oder Carbonsäureanhydriden in Mengen von 0,01 bis 20 Gew.-%, bezogen auf eingesetzte Fettsäurealkylester einer Temperaturbehandlung oberhalb von 150°C für einen Zeitraum bis zu 60 Minuten unterwirft und gleichzeitig und/oder anschliessend die gereinigten Fettsäurealkylester vom behandelten Gut abtrennt oder

b) freie Fettsäuren pflanzlichen und/oder tierischen Ursprungs in Gegenwart von Veresterungskatalysatoren und/oder Carbonsäureanhydriden in Mengen von 0,01 bis 20 Gew.-%, bezogen auf eingesetzte Fettsäuren, einer Temperaturbehandlung oberhalb von 150°C für einen Zeitraum bis zu 60 Minuten unterwirft, gleichzeitig und/oder anschliessend die gereinigten Fettsäuren abtrennt und die abgetrennten Fettsäuren in bekannter Weise mit Alkoholen verestert.

2. Verfahren nach Anspruch 1, dadurch gekennzeichnet, dass man das Einsatzmaterial bei Temperaturen im Bereich von 150 bis 280°C, bevorzugt im Temperaturbereich von 200 bis 250°C behandelt.

3. Verfahren nach Ansprüchen 1 und 2 dadurch gekennzeichnet, dass man die Temperaturbehandlung für einen Zeitraum nicht über 30 Minuten und bevorzugt bei Verweilzeiten von 0,1 bis 15 Minuten durchführt.

4. Verfahren nach Ansprüchen 1 bis 3, dadurch gekennzeichnet, dass man Temperatur- und Verweilzeit derart aufeinander abstimmt, dass eine Veresterung bzw. Umesterung freier Hydroxylgruppen stattfindet, ohne dass im übrigen die Struktur der Fettsäurealkylester bzw. Fettsäuren substantiell verändert wird.

5. Verfahren nach Anspruch 4, dadurch gekennzeichnet, dass mit Verweilzeiten von 0,1 bis 3 Minuten im Temperaturbereich von 200 bis 250°C gearbeitet wird.

6. Verfahren nach Ansprüchen 1 bis 5, dadurch gekennzeichnet, dass unter vermindertem Druck, bevorzugt im Druckbereich von 0,13 bis 13,3 mbar (0,1 bis 10 Torr) gearbeitet wird.

7. Verfahren nach Ansprüchen 1 bis 6, dadurch gekennzeichnet, dass Raffination und Abtrennung der gereinigten Fettsäurealkylester bzw. Fettsäuren durch Vakuumdestillation zu einer kontinuierlich betriebenen Verfahrensstufe zusammengefasst werden.

8. Verfahren nach Ansprüchen 1 bis 7, dadurch gekennzeichnet, dass mit Veresterungskatalysatoren gearbeitet wird, die im Einsatzmaterial und/oder in den bei der Raffination gebildeten Hochsiedern löslich sind.

9. Verfahren nach Ansprüchen 1 bis 8, dadurch gekennzeichnet, dass mit alkalischen oder neutralen Veresterungskatalysatoren gearbeitet wird.

10. Verfahren nach Ansprüchen 1 bis 9, dadurch gekennzeichnet, dass man in Gegenwart hochsiedender Carbonsäureanhydride arbeitet, die oberhalb der zu raffinierenden Fettsäurealkylester bzw. Fettsäuren sieden.

11. Verfahren nach Ansprüchen 1 bis 10, dadurch gekennzeichnet, dass man die Veresterungskatalysatoren und/oder die Carbonsäureanhydride (reaktive Hilfsstoffe) dem zu behandelnden Gut in einer Menge von 0,01 bis 10 Gew.-% bevorzugt in Mengen nicht über 5 Gew.-% − bezogen auf eingesetzte Fettsäurealkylester bzw. Fettsäuren − zusetzt.

12. Verfahren nach Ansprüchen 1 bis 11, dadurch gekennzeichnet, dass man gehärtete oder ungehärtete Fettsäurealkylester- bzw. Fettsäuregemische aus natürlichen pflanzlichen und/oder tierischen Fetten dem Verfahren unterwirft.

## Claims

1. A process for the production of fatty acid alkyl esters having improved processability, characterized in that

a) alkyl esters of fatty acids of vegetable and/or animal origin are subjected to a heat treatment above 150 °C for up to 60 minutes in the presence of esterification catalysts and/or carboxylic acid anhydrides in quantities of from 0.01 to 20% by weight, based on the fatty acid alkyl esters used, and the purified fatty acid alkyl esters are simultaneously and/or subsequently separated off from the treated material, or

b) free fatty acids of vegetable and/or animal origin are subjected to a heat treatment above 150 °C for up to 60 minutes in the presence of esterification catalysts and/or carboxylic acid anhidrics in quantities of from 0.01 to 20% by weight, based on the fatty acids used, the purified fatty acids are simultaneously and/or subsequently separated off and the fatty acids separated off are esterified with alcohols in known manner.

2. A process as claimed in Claim 1, characterized in that the starting material is treated at temperatures of from 150 to 280 °C and preferably at temperatures of from 200 t0 250 °C.

3. A process as claimed in Claims 1 and 2, characterized in that the heat treatment is carried out for no longer than 30 minutes and preferably for 0.1 to 15 minutes.

4. A process as claimed in Claims 1 to 3, characterized in that the treatment temperature and treatment time are coordinated with one another in such a way that free hydroxyl groups are esterified or transesterified without the remaining structure of the fatty acid alkyl esters or fatty acids being substantially affected.

5. A process as claimed in Claim 4, characterized in that the heat treatment is carried out for 0.1 to 3 minutes at a temperature of from 200 to 250 °C.

6. A process as claimed in Claims 1 to 5, characterized in that the heat treatment is carried out under reduced pressure and preferably under a pressure of from 0.13 to 13.3 mbar (0.1 to 10 Torr).

7. A process as claimed in Claims 1 to 6, characterized in that refining and separation of the purified fatty acid alkyl esters or fatty acids by vacuum distillation are combined into a continuous process step.

8. A process as claimed in Claims 1 to 7, characterized in that the esterification catalysts used are soluble in the starting material and/or in the high-boiling fractions formed during refining.

9. A process as claimed in Claims 1 to 8, characterized in that alkaline or neutral esterification catalysts are used.

10. A process as claimed in Claims 1 to 9, characterized in that the heat treatment is carried out in the presence of carboxylic acid anhydrides which boil above the fatty acid alkyl esters or fatty acids to be refined.

11. A process as claimed in Claims 1 to 10, characterized in that the esterification catalysts and/or the carboxylic acid anhydrides (reactive auxiliaries) are added to the material to be treated in a quantity of from 0.01 to 10% by weight and preferably in a quantity of no more than 5% by weight, based on the fatty acid alkyl esters or fatty acids used.

12. A process as claimed in Claims 1 to 11, characterized in that hardened or unhardened fatty acid alkyl ester or fatty acid mixtures of natural vegetable and/or animals fats are subjected to the process.

## Revendications

1. Procédé de fabrication d'esters alcoylés d'acides gras se travaillant mieux, caractérisé en ce que

a) on soumet des esters alcoylés d'acides gras d'origine végétale et/ou animale à un traitement thermique au-dessus de 150 °C pendant un laps de temps allant jusqu'à 60 minutes en présence de catalyseurs d'estérification et/ou d'anhydrides d'acides carboxyliques en des quantités de 0,01 à 20% en poids par rapport à l'ester alcoylé d'acide gras mis en jeu et en ce qu'en même temps et/ou consécutivement on sépare les esters alcoylés d'acides gras purifiés à partir de la matière traitée, ou

b) on soumet des acides gras libres d'origine végétale et/ou animale à un traitement thermique au-dessus de 150 °C pendant un laps de temps allant jusqu'à 60 minutes en présence de catalyseurs d'estérification et/ou d'anhydrides d'acides carboxyliques en des quantités de 0,01 à 20% en poids par rapport aux acides gras mis en jeu et en ce qu'en même temps et/ou consécutivement on sépare les acides gras purifiés et estérifie les acides gras séparés avec des alcools, de façon connue.

2. Procédé selon la revendication 1, caractérisé en ce qu'on traite la matière chargée à des températures dans l'intervalle de 150 à 280 °C, de préférence dans l'intervalle de température de 200 à 250 °C.

3. Procédé selon les revendications 1 et 2, caractérisé en ce qu'on exécute le traitement thermique pendant un laps de temps non supérieur à 30 minutes et de préférence avec des temps de séjour de 0,1 à 15 minutes.

4. Procédé selon les revendications 1 à 3, caractérisé en ce qu'on accorde mutuellement la température et le temps de séjour pour que se produise une estérification ou transestérification des groupes hydroxyle libres, sans que du reste la structure des esters alcoylés d'acides gras ou des acides gras soit substantiellement modifiée.

5. Procédé selon la revendication 4, caractérisé en ce qu'on opère avec des temps de séjour de 0,1 à 3 minutes dans l'intervalle de température de 200 à 250 °C.

6. Procédé selon les revendications 1 à 5, caractérisé en ce qu'on opère sous pression réduite, de préférence dans l'intervalle de pression de 0,13 à 13,3 mbars (0,1 à 10 torrs).

7. Procédé selon les revendications 1 à 6, caractérisé en ce qu'on associe le raffinage à la séparation des esters alcoylés d'acides gras ou des acides gras purifiés par une distillation sous vide en un stade opératoire exécuté en continu.

8. Procédé selon les revendications 1 à 7, caractérisé en ce qu'on opère avec des catalyseurs d'estérification qui sont solubles dans la matière chargée et/ou dans les substances à point d'ébullition élevé formées au cours du raffinage.

9. Procédé selon les revendications 1 à 8, caractérisé en ce qu'on opère avec des catalyseurs d'estérification alcalins ou neutres.

10. Procédé selon les revendications 1 à 9, caractérisé en ce qu'on opère en présence d'anhydrides d'acides carboxyliques à point d'ébullition élevé, qui bouillent plus haut que les esters alcoylés d'acides gras ou les acides gras devant être raffinés.

11. Procédé selon les revendications 1 à 10, caractérisé en ce qu'on ajoute les catalyseurs d'estérification et/ou les anhydrides d'acides carboxyliques (auxiliaires réactifs) à la matière à traiter en une quantité de 0,01 à 10% en poids, de préférence en des quantités non supérieures à 5% en poids par rapport aux esters alcoylés d'acides gras ou aux acides gras mis en jeu.

12. Procédé selon les revendications 1 à 11, caractérisé en ce qu'on soumet au procédé des mélanges d'esters alcoylés d'acides ou d'acides gras durcis ou non durcis en provenance de graisses naturelles végétales et/ou animales.